# EUROPEAN PATENT SPECIFICATION

(11) **EP 4 190 286 B1**
(45) Date of publication and mention of the grant of the patent: **18.03.2026**
(21) Application number: 22211414.2
(22) Date of filing: 05.12.2022
(51) Int. Cl.: A61F 9/008, A61F 9/009

(54) **OPHTALMOLOGICAL TREATMENT AND MANUFACTURE APPARATUS**
OPHTALMOLOGISCHE BEHANDLUNGS- UND HERSTELLUNGSVORRICHTUNG
APPAREIL DE TRAITEMENT ET DE FABRICATION OPHTALMOLOGIQUE

(30) Priority: 06.12.2021 CH 0706552021
(43) Date of publication of application: 07.06.2023
(73) Proprietor: Ziemer Ophthalmic Systems AG, 2562 Port (CH)
(72) Inventor: MARASHI, Mohamad Omar, Aleppo (SY)
(74) Representative: Rentsch Partner AG

(56) References cited:
- WO-A1-2004/017878
- IT-A1- 201800 009 697
- US-A1- 2008 058 777
- US-A1- 2008 082 086
- US-A1- 2019 159 934

## Description

### TECHNICAL FIELD

The present invention relates to the field of ophthalmological surgery, in particular posterior lamellar keratoplasty. Specifically, it relates to ophthalmological treatment apparatus and ophthalmological manufacture apparatuses, as well related methods. The invention further relates to cornea implants.

### BACKGROUND, PRIOR ART

In posterior lamellar keratoplasty, a pathological respectively defective patient cornea element of a patient cornea is resected and is replaced with a cornea implant which may be manufactured from a donor cornea. The patient cornea element and accordingly the cornea implant are adjacent to the anterior chamber. For resecting the patient cornea element as well as manufacturing the cornea implant a suited surgical, in particular ophthalmological, laser device.

### SUMMARY OF DISCLOSURE

A number of apparatuses and procedures for posterior lamellar keratoplasty are generally known in the art, for example from US2008/0082086A1. The involved procedures, however, are generally highly complex and critical, with a substantive risk for damaging the patient's eye. This particularly concerns the removal of the resected patient cornea element as well as the insertion and positioning of the tissue implant. Further there is a general risk for the resected patient cornea element as well as the cornea implant to fall into the anterior chamber of the patient eye. Also, correct positioning of the cornea implant without undesired movement thereof subsequent to insertion is critical.

WO2008/030699A2 describes an approach where annular incisions and side cuts are impended in the donor as well as the patient cornea tissue to allow securing of the cornea implant. The resulting geometry, however, is complex and actually inserting and positioning the cornea implant difficult.

It is an overall objective of the present disclosure to improve the state of the art regarding posterior lamellar keratoplasty. Favorably, at least some of the before-mentioned drawbacks of the prior art are reduced and favorably avoided.

In an aspect, the present disclosure concerns an ophthalmological treatment apparatus for resecting a patient cornea element from a patient cornea in posterior lamellar keratoplasty. The ophthalmological treatment apparatus includes a treatment laser device for implementing cornea cuts. The ophthalmological treatment apparatus further includes a computerized treatment control device in operative coupling with the treatment laser device, the treatment control device being designed to control the treatment laser device to implement a number of patient cornea cuts. The patient cornea cuts form, in combination, a patient cornea cut pattern. A geometry of the patient cornea element is defined by the patient cornea cut pattern.

The number of patient cornea cuts include a patient anterior lamellar cut. The patient anterior lamellar cut extends in the patient cornea at a patient anterior lamellar cut distance from a patient posterior cornea surface. The patient anterior lamellar cut defines a patient anterior cornea element surface. The patient posterior cornea element surface is adjacent to the anterior surface of the patient eye.

The number of patient cornea cuts may further include a patient side cut. The patient side cut extends in the patient cornea from a periphery, in particular a circumferential edge, of the patient anterior lamellar cut towards the patient posterior cornea surface in a circumferentially closed manner. The patient side cut defines, at least in part, a circumferential patient cornea element surface.

The number of patient cornea cuts may further include a patient access cut. The patient access cut extends in the patient cornea from the patient side cut a to a peripheral region of the patient anterior cornea surface. The patient access cut forms an access passage in the patient cornea.

The patient cornea cut pattern may further define a patient cornea interlocking structure. The patient cornea interlocking structure is configured to interlock the patient cornea with a cornea implant after replacing the patient cornea element by the cornea implant.

In a further aspect, the present disclosure concerns an ophthalmological manufacture apparatus for manufacturing from a donor cornea a cornea implant. The manufactured cornea implant is suited for use in in posterior lamellar keratoplasty, the ophthalmological manufacture apparatus may include a manufacture laser device for implementing cornea cuts. The ophthalmological manufacture apparatus may further include a computerized manufacture control device in operative coupling with the manufacture laser device. The manufacture control device is designed to control the manufacture laser device to implement a number of donor cornea cuts. The donor cornea cuts form, in combination, a donor cornea cut pattern. A geometry of the cornea implant is defined by the donor cornea cut pattern.

The number of donor cornea cuts includes a donor anterior lamellar cut. The donor anterior lamellar cut extends in the donor cornea at a donor anterior lamellar cut distance from a donor posterior cornea surface of the donor cornea, the donor anterior lamellar cut defining a cornea implant anterior surface. A cornea implant posterior surface is generally defined by the portion of the donor posterior conceal surface which is delimited respectively circumscribed by the donor side cut.

The number of donor cornea cuts further includes a donor side cut. The donor side cut extends in the donor cornea from the donor anterior lamellar cut towards the donor posterior cornea surface in a circumferentially closed manner. A junction of the donor side cut and the donor anterior lamellar cut is displaced with respect to a circumferential edge, of the donor anterior lamellar cut. The donor side cut defines, at least in part, a circumferential cornea implant surface.

The number of donor cornea cuts may further include a donor access cut. The donor access cut extends from a periphery, in particular a circumferential edge, of the donor anterior lamellar cut to a donor anterior cornea surface.

The donor cornea cut pattern may further define a cornea implant interlocking structure. The cornea implant interlocking structure is configured to interlock the patient cornea with the cornea implant after replacing the patient cornea element by the cornea implant.

In a further aspect, the present disclosure concerns a method for manufacturing from a donor cornea a cornea implant suited for use in in posterior lamellar keratoplasty, in particular using an ophthalmological manufacture apparatus according in accordance with the present disclosure. The method may include implementing by means of a laser device a number of donor cornea cuts. The donor cornea cuts form, in combination, a donor cornea cut pattern. A geometry of the cornea implant may be defined by the donor cornea cut pattern. The laser device may in particular be a manufacture laser device of a manufacture apparatus in accordance with the present disclosure. In accordance with the claims, the method is not being practiced on the living human or animal body.

The number of donor cornea cuts includes a donor anterior lamellar cut. The donor anterior lamellar cut extends in the donor cornea at a donor anterior lamellar cut distance from a donor posterior cornea surface. The donor anterior lamellar cut defines a cornea implant anterior surface.

The number of donor cornea cuts further includes a donor side cut. The donor side cut extends in the donor cornea from the donor anterior lamellar cut towards the donor posterior cornea surface in a circumferentially closed manner. A junction of the donor side cut and the donor anterior lamellar cut is displaced with respect to a circumferential edge, of the donor anterior lamellar cut. The donor side cut defines at least in part, a circumferential cornea implant surface.

The number of donor cornea cuts further includes a donor access cut. The donor access cut extends from a periphery, in particular a circumferential edge, the circumferential edge of the donor anterior lamellar cut to a donor anterior cornea surface.

The donor cornea cut pattern may further define a cornea implant interlocking structure. The cornea implant interlocking structure is configured to interlock a patient cornea with the cornea implant after replacing the patient cornea element by the cornea implant. In a further not claimed aspect, the present disclosure concerns an ophthalmological surgical method for replacing a patient cornea element with a cornea implant by way of posterior lamellar keratoplasty. The method may in particular be carried out using an ophthalmological treatment apparatus in accordance with the present disclosure.

Said ophthalmological surgical method includes implementing by means of a laser device, in particular an ophthalmological laser device, a number of patient cornea cuts. The patient cornea cuts form, in combination, a patient cornea cut pattern. A geometry of the patient cornea element is defined by the patient cornea cut pattern. The laser device may be a treatment laser device and/or ophthalmological laser device in accordance with the present disclosure.

The number of patient cornea cuts includes cuts as described above in the context of an ophthalmological treatment apparatus above as well as particular embodiments of such apparatus as discussed further below.

Said ophthalmological surgical method includes, subsequent to resecting the patient cornea element respectively implementing the patient cornea cuts, removing the patient cornea element from the patient cornea, in particular via the patient access, cut, thereby creating a patient cornea cavity in the patient cornea. Removing the patient cornea element may include inserting forceps into patient access cut and grasping the patient cornea element.

Said ophthalmological surgical method includes placing the cornea implant in the patient cornea cavity, in particular via the patient access cut, in particular using for example an insertion spatula.

It is noted that the patient cornea element and the corresponding patient cornea cavity as well as the cornea implant are of generally the same shape and generally identical dimensions.

Said ophthalmological surgical method further includes interlocking the cornea implant with the patient cornea by engagement of a patient cornea interlocking structure of the patient cornea with a cornea implant interlocking structure of the cornea implant.

In a further as such not claimed aspect, the present disclosure concerns a cornea implant. The cornea implant may in particular be manufactured using an ophthalmological manufacture apparatus in accordance with the present disclosure and/or a manufacture method in accordance with the present disclosure. In an embodiment, a cornea implant in accordance with the present disclosure is made from tissue of a donor cornea.

The cornea implant includes a curved, in particular spherically curved, cornea implant anterior surface. The cornea implant further includes a curved cornea implant posterior surface which is displaced with respect to the cornea implant anterior surface in a posterior direction.

The cornea implant further includes a circumferential cornea implant surface. The circumferential cornea implant surface extends between a periphery, in particular a circumferential edge, of the cornea implant anterior surface and a periphery, in particular a circumferential edge, of the cornea implant posterior surface.

The cornea implant further includes a cornea implant interlocking structure. The cornea implant interlocking structure is configured to interlock a patient cornea with the cornea implant after replacing a patient cornea element of a patient cornea with the cornea implant. The cornea implant interlocking structure may in an embodiment be arranged at and/or forms part of the cornea implant posterior surface.

The patient cornea element is the piece of the patient cornea that shall be replaced by a cornea implant in posterior lamellar keratoplasty. Posterior lamellar keratoplasty in accordance with the present disclosure is particularly favorable since the patient access cut allows an easy removal of the patient cornea element and subsequent insertion of the cornea implant in a comparatively easy and safe manner with minimal risk of causing unintentional damage of the cornea as well as complications in general. By the patient access cut extending to the peripheral region of the patient anterior cornea surface, the patient cornea element can be pulled out, e.g. using forceps, in a substantially linear movement and without exerting excessive stress on the tissue of the cornea. After removal of the resected patient cornea element, a patient cornea cavity is present in the patient cornea with a shape that generally corresponds to the patient cornea element. A cornea implant of generally the same shape may subsequently be inserted respectively placed in the patient cornea cavity in the same way, i.e. via the patient access cut.

A further advantage of posterior lamellar keratoplasty in accordance the present disclosure is in some embodiments the provision of a cornea implant at the cornea implant posterior surface. The patient cornea interlocking structure may be complementary to the cornea implant interlocking structure, thereby allowing a form fit Such interlocking structure allows efficient and safe establishment of an interlocking between cornea implant and patient cornea and in particular prevents the resected patient cornea element as well as the cornea implant from falling into the adjacent anterior chamber of the patient eye during manipulation. As will be explained in more detail below, the patient cornea interlocking structure supports the patient cornea element after implementing the patient cornea cuts before it is removed, and further supports the cornea implant upon being placed in the patient cornea cavity. Also, posterior lamellar keratoplasty in accordance the present disclosure avoids the need for sutures. Further advantages of particular embodiments are discussed further below in their respective context.

The patient anterior lamellar cut is defined by a closed and spatially curved, for example spherically curved, cut surface that is delimited by a circumferential edge. Typically, a patient cornea axis extends through a center of the patient anterior lamellar cut as axis of rotational symmetry. The patient anterior lamellar cut is generally smooth. For the patient anterior lamellar cut being spherically curved, the circumferential edge of the patient anterior lamellar cut is generally circular.

The patient cornea axis is generally a central axis respectively axis of symmetry of the patient cornea and of the patient cornea element that is resected. It generally corresponds to the optical axis of the patient eye. Similarly, the donor cornea axis is a central axis respectively axis of symmetry of the donor cornea and corresponds to the cornea implant axis of a cornea implant manufactured from the donor cornea. In a viewing direction transverse to the respective axis, the patient cornea element and the cornea implant are generally circular or substantially circular.

A patient posterior cornea element surface is generally defined by the portion of the patient posterior cornea surface which is delimited respectively circumscribed by the patient side cut. In an embodiment where the circumferential edge of the patient anterior lamellar cut is circular as mentioned, the patient side cut may for example have the shape of a tubular ring that may extend parallel to the patient cornea axis. Designs, however, are also possible, e.g. with the circumferential edge of the patient anterior lamellar cut being angled with respect to the patient cornea axis. The same applies in an analog manner for the donor and the cornea implant.

The patient anterior cornea element surface and the patient posterior cornea element surface (forming part of the patient posterior cornea surface) are generally spaced apart with respect to each other along the patient cornea axis. Similarly, the cornea implant anterior surface and the cornea implant posterior surface (forming part of the donor posterior cornea surface when manufactured from a donor cornea) are generally spaced apart with respect to each other along the cornea implant axis respectively the donor cornea axis.

The patient anterior lamellar cut and the donor anterior lamellar cut are generally spatially curved, in particular in a spherically curved, with the patient cornea axis respectively donor cornea axis as axis of symmetry. The same applies, in an analogue manner, to the donor and to the cornea implant.

In dependence of the desired shape of the cornea implant, the patient anterior lamellar cut distance may be constant over the patient anterior lamellar cut, or may vary over the patient anterior lamellar cut. It may in particular vary in a radial manner with respect to the patient cornea axis. The same applies in an analogue manner for the donor anterior lamellar cut distance.

As mentioned before, the shape and dimensions of the patient cornea element, respectively the patient cornea cavity after removal of the patient cornea element generally correspond to the shape and geometry of the cornea implant and the cornea cuts may be implemented accordingly in the donor cornea and the patient cornea. In particular, the patient anterior lamellar cut may be identical or substantially identical to the donor anterior lamellar cut, and the patient side cut may be identical or substantially identical to the donor side cut. Similar, a patient posterior lamellar cut as present in an embodiment as discussed further below may be identical or substantially identical to a donor posterior lamellar cut. A patient auxiliary side cut as present in an embodiment as discussed further below may be identical or substantially identical to a donor auxiliary side cut.

However, some deviation may be present between patient and donor cuts in dependence of the specific situation. By way of example, a donor auxiliary side cut may have an extension that slightly differs from the corresponding patient auxiliary side cut. Similarly, a donor posterior lamellar cut may somewhat differ from the patient posterior lamellar cut, and/or donor side cut may somewhat differ from the patient side cut. A donor cut may in particular be longer respectively wider or narrower respectively shorter as comparted to the corresponding patient cut. Such intentional differences between donor cut and side cuts may be used to avoid interference between tissue implant and patient cornea as well as to ensure correct positioning and fitting.

The patient access cut may meet the patient side cut at the junction of the patient side cut and the patient anterior lamellar cut. The patient access cut, however, may also meet the patient side cut displaced in posterior direction with respect to the junction of the patient side cut and the patient anterior lamellar cut.

Further, the patient cornea interlocking structure and cornea implant interlocking structure are generally complementary to each other. In an embodiment, the patient cornea interlocking structure is defined, at least in part, by a patient posterior cornea element surface. Such embodiments are discussed further below in more detail.

Alternatively, or additionally however, the interlocking structures may be arranged fully or partly at the circumference respectively circumferential surface of the patient cornea cavity and the cornea implant, respectively. In particular, the circumference surface of the patient cornea cavity and the cornea implant may include complementary positive respectively convex and negative respectively concave interlocking features that are designed for mutual engagement. Positive interlocking features may, for example, be formed by protrusions such as circumferential rims or tabs, and negative interlocking features may be formed by recesses or cutouts. Further the circumferential surface of the patient cornea cavity and the cornea implant may extend between proximal and distal with complimentary zig-zag shapes. In embodiments where the interlocking structures are circumferentially arranged, the patient side cut and the donor side cut are generally no smooth and continuous cuts, but are segmented in order to provide the patient cornea cavity interlocking structure and cornea implant interlocking structure.

In an embodiment, the treatment control device may be configured to receive patient cut information, the patient cut information defining the patient cornea cut pattern. Similarly, a manufacture control device may be may be configured to receive donor cut information, the patient cut information defining the donor cornea cut pattern.

The patient cut information and/or donor cut information may be received, for example, via a data interface from a further device or on a data carrier, such as a CD-ROM, or may be acquired by an ophthalmologic measurement device in operative coupling with the treatment control device or manufacture control device, respectively an ophthalmological manufacture and treatment control device.

According to a further aspect, the present disclosure concerns a computer program product including a non-transient computer readable medium having stored therein computer program code. The computer program code is configured to direct a processor of a computerized treatment control device of an ophthalmological treatment apparatus to control a treatment laser device of the ophthalmological treatment apparatus to implement a number of patient cornea cuts, the patient cornea cuts forming, in combination, a patient cornea cut pattern. The number of patient cornea cuts respectively the patient cornea cut pattern may correspond to any embodiment as discussed above and/or further below in the context of an ophthalmological treatment apparatus and/or ophthalmological manufacture and treatment apparatus.

According to a further aspect, the present disclosure concerns a further computer program product including a non-transient computer readable medium having stored therein computer program code. The computer program code is configured to direct a processor of a computerized manufacture control device of an ophthalmological manufacture apparatus to control a manufacture laser device of the ophthalmological manufacture apparatus to implement a number of donor cornea cuts, the donor cornea cuts forming, in combination, a donor cornea cut pattern. The number of donor cornea cuts respectively the donor cornea cut pattern may correspond to any embodiment as discussed above and/or further below in the context of an ophthalmological manufacture apparatus and/or ophthalmological manufacture and treatment apparatus.

In an embodiment, the treatment control device is configured to control the treatment laser device to implement the patient anterior lamellar cut subsequent to the patient side cut and the patient access cut subsequent to the patient anterior lamellar cut. Also, in an embodiment, the manufacture control device is configured to implement the donor anterior lamellar cut subsequent to the donor side cut and the donor access cut subsequent to the donor anterior lamellar cut.

In accordance with the claims, the patient side cut does not extend to the patient posterior cornea surface. Further, the patient cornea cut pattern includes a patient posterior lamellar cut. The patient posterior lamellar cut extends circumferential in the patient cornea from a posterior end, in particular a posterior edge, of the patient side cut inwards with respect to the patient cornea, in particular with a constant distance to the patient anterior lamellar cut. The patient cornea cut pattern furthers include a patient auxiliary side cut. The patient auxiliary side cut extends from an inner periphery, in particular an inner edge, of the patient posterior lamellar cut to the patient posterior cornea surface.

In accordance with the claims, the donor side cut does not extend to the donor posterior cornea surface. The donor cornea cut pattern further includes a donor posterior lamellar cut. The donor posterior lamellar extends in the donor cornea from a posterior edge of the donor side cut inwards with respect to the donor cornea, in particular with a constant distance to the donor anterior lamellar cut. The donor cornea cut pattern further includes a donor auxiliary side cut. The donor auxiliary side cut extends from an inner edge of the donor posterior lamellar cut to the donor posterior cornea surface.

It is noted that the patient cornea element and the corresponding patient cornea cavity and the cornea implant are of generally the same shape and generally identical dimensions. Therefore, considerations and embodiments for the patient cornea cut pattern, in particular a patient posterior lamellar cut and patient auxiliary side cut generally hold true for the donor cornea cut pattern, in particular a donor posterior lamellar cut and donor auxiliary side cut, in an analogue manner.

In an embodiment with a patient posterior lamellar cut, a part of the patient cornea element that is delimited in posterior direction by the patient posterior lamellar cut is referred to as peripheral part, while the part that is in posterior direction delimited by the patient posterior cornea surface is referred to as inner or central part. Generally, the peripheral part of the patient cornea element circumferentially surrounds the inner or central part. The same applies in an analog manner for the donor and the cornea implant.

In an embodiment, the patient posterior lamellar cut is shaped in the same or substantially the same manner as the patient anterior lamellar cut and may, e.g., also be spherical. Further, the patient posterior lamellar cut may extend with a generally uniform respectively constant distance to the patient anterior lamellar cut. In alternative embodiments, the patient posterior lamellar cut may e.g., be planar and extend either transverse, in particular normal, or oblique to the patient cornea axis. For an oblique patient posterior lamellar cut, the peripheral part of the patient cornea element and accordingly also the cornea implant may be tapered respectively frustoconical and be thicker at the periphery as comparted to the transition to the inner or central part. Further designs of the patient posterior cornea surface apart from planar or spherical are possible as well.

In an embodiment, the patient cornea element includes a circumferential step in a patient posterior cornea element surface, in particular in a peripheral region of the patient posterior cornea element surface. In an embodiment of the cornea implant, the cornea implant posterior surface includes a circumferential step in a cornea implant posterior surface, in particular in a peripheral region of the cornea implant posterior surface. As will be explained in the following, such embodiments are particularly favorable for interlocking the cornea implant with respect to the patient cornea by way of complementary structures.

The patient posterior lamellar cut and the patient auxiliary side cut result for such type of embodiment in a peripheral step being formed in the patient cornea element at the patient posterior cornea element surface. Similarly, the donor posterior lamellar cut and the donor auxiliary side cut result in a peripheral step being formed in the cornea implant at the cornea implant posterior surface. The step may in particular include a circumferential recess that extends laterally respectively radially from the patient auxiliary side cut to the circumferential or outer edge of the patient cornea element, respectively from the donor auxiliary side cut to the circumferential or outer edge of the cornea implant. The patient posterior lamellar cut and the donor posterior lamellar cut may in particular be annular cuts.

For such embodiment, a central part of the patient posterior cornea element surface around the patient cornea axis may directly formed by the patient posterior cornea surface as is was prior to implementing the patient cornea cuts. A peripheral part of the patient posterior cornea element surface is defined by the patient posterior annular cut. Similarly, a central part of the cornea implant posterior surface around the donor cornea axis respectively the cornea implant axis may be directly formed by the donor posterior cornea surface as is was prior to implementing the donor cornea cuts. A peripheral part of the cornea implant posterior surface may be defined by the donor posterior lamellar cut. The central and the peripheral part of the patient posterior cornea element surface and the cornea implant posterior surface are separated via the patient auxiliary side cut respectively the donor auxiliary side cut.

The thickness of the patient cornea element respectively the distance between the patient anterior cornea element surface and the patient posterior cornea element surface may be larger for the inner or central part of the patient cornea element as compared to the outer or peripheral part. Similarly, the thickness of the cornea implant respectively the distance between the cornea implant anterior surface and the cornea implant posterior surface may be larger for the inner or central part of the cornea implant as compared to the outer or peripheral part.

For such embodiment, a lateral respectively radial extension of the step or recess in the patient cornea element is determined by respectively corresponds to the patient posterior lamellar cut, while a longitudinal extension of the step or recess in the patient cornea element is determined by respectively corresponds to the patient auxiliary side cut. Similarly, a lateral respectively radial extension of the step or recess in the cornea implant is determined by respectively corresponds to the donor posterior lamellar cut, while a longitudinal extension of the step or recess in the cornea implant is determined by respectively corresponds to the donor auxiliary side cut.

After removing the patient cornea element from the patient cornea in the before-explained type of embodiment, a patient cornea tissue ring will extend into the patient cornea cavity at the posterior side of the patient cornea cavity at the boarder to the anterior chamber. The thickness of the patient cornea tissue ring is determined respectively corresponds to the extension respectively length of the patient auxiliary side cut, while its radial respectively lateral extension is determined by respectively corresponds to the extension of the patient posterior lamellar cut. When placing the cornea implant in the patient cornea cavity, the cornea implant interlocking structure, in particular a recess as defined for example by a posterior lamellar cut and donor auxiliary side cut as mentioned before engages the patient cornea tissue ring, thereby interlocking the cornea implant with the patient cornea and in particular preventing the cornea implant from falling into the anterior chamber. When the cornea implant is positioned, the peripheral part of the cornea implant posterior surface contacts respectively abuts the anterior surface of the patient cornea tissue ring and the cornea implant rests on the patient cornea tissue ring.

Where the patient posterior lamellar cut extends in a constant distance to the patient anterior lamellar cut, it generally has a corresponding contour and may, for example be spherically curved. Similarly, where the donor posterior lamellar cut extends in a constant distance to the donor anterior lamellar cut, it generally has a corresponding contour. In alternative embodiment, however, the patient posterior lamellar cut and the donor posterior lamellar cut may in each case have a contour that differs from the contour of the respective anterior cut. Bay way of example, the patient posterior lamellar cut and the donor posterior lamellar cut may be flat respectively planar. In a further embodiment, the patient posterior annular cut may for example be corrugated. In any case, the contour of the patient posterior lamellar cut and the donor posterior lamellar may have an identical or substantially identical contour, resulting in the peripheral portion of the posterior cornea implant matching respectively being complementary to the anterior surface of the patient cornea tissue ring, thereby ensuring an aerial contact respectively a contact over their full surface.

In an embodiment, the treatment control device is configured to control the treatment laser device to implement the patient posterior lamellar cut subsequent to the patient auxiliary side cut and prior to the patient side cut. This type of embodiment is favorable regarding an efficient and precise implementation of the patient cornea cuts. Similarly, the manufacture control device may be configured to control the manufacture laser device to implement the donor posterior lamellar cut subsequent to the donor auxiliary side cut and prior to the donor side cut.

In an embodiment, the patient access cut meets the patient anterior cornea surface in an acute angle. Such arrangement is favorable for removing the patient cornea element and subsequently inserting the cornea implant I a smooth manner and a shallow angle with respect to the patient anterior cornea surface.

In an embodiment, the patient cornea cut pattern includes an auxiliary patient access cut. The auxiliary patient access cut extends in the patient cornea from the patient side cut to the peripheral region of the patient anterior cornea surface. The auxiliary patient access cut is arranged with respect to a circumference of the patient side cut opposite to the patient access cut. The auxiliary patient access cut forms an auxiliary access passage in the patient cornea. In a view along the patient cornea axis, the auxiliary patient access cut and the patient access cut may be diametrically opposed to each other. In an embodiment, the patient auxiliary access cut meets the patient anterior cornea surface in a perpendicular or substantially perpendicular manner.

The auxiliary patient access cut may meet the patient side cut at the posterior edge of the patient side cut, respectively in some embodiments a mentioned before at the junction of the patient side cut and the patient posterior lamellar cut. The auxiliary patient access cut, however, may also meet the patient side cut displaced in anterior direction respectively displaced in direction towards the patient anterior lamellar cut with respect to the posterior edge of the patient side cut.

Providing an auxiliary patient access cut in addition to the patient access cut as mentioned before is favorable regarding the insertion and positioning of the cornea implant in the patient cornea cavity. Subsequent to placing the cornea implant in the patient cornea cavity as mentioned before, the cornea implant may additionally be grasped by a favorably serrated auxiliary forceps that is inserted into the auxiliary patient access cut. By maneuvering the cornea implant with an insertion spatula as well as the auxiliary forceps, it may be placed in the correct position. In particular, the auxiliary forceps may be used to the pull the patient cornea implant into position.

In an embodiment, the treatment control device is configured to the patient the patient cornea cuts that define the geometry of the patient cornea element in sequence one after the other. Similarly, the manufacture control device may be configured to implement the donor cornea cuts that define the geometry of the cornea implant in sequence one after the other. These types of embodiments are particularly favorable regarding precision and efficiency. In particular, the end of each of such cuts corresponds at the same time to the beginning of a next following cut.

In any case, the patient cornea cuts as well as the tissue cornea cuts are implemented in an order and with cutting direction that ensures that no shading occurs when implementing any cut as a result from previous cuts. This generally implies an implementation of the cuts from posterior (first) to anterior.

Regarding the ophthalmological treatment apparatus and the ophthalmological manufacture apparatus, it is noted that they may be different and distinct apparatuses which may also be present at different locations. Alternatively, however, they may be partly or fully integrated as combined ophthalmological manufacture and treatment apparatus. The computerized treatment control device may be integral with a computerized manufacture control device, respectively an ophthalmological manufacture and treatment control device may be both a computerized treatment control device and a computerized manufacture control device.

Similarly, the treatment laser device may be integral with a manufacture laser device, respectively an ophthalmological laser device may be both a treatment laser device and a manufacture laser device.

Where not explicitly stated differently, expressions related to components of an apparatus may refer to an ophthalmological treatment apparatus, an ophthalmological manufacture apparatus or an ophthalmological manufacture and treatment apparatus. In particular, the expression "control device" may generally refer to a treatment control device, a manufacture control device or an ophthalmological manufacture and treatment control device as mentioned. Similarly, the expression "ophthalmological laser device" may refer to a treatment laser device, manufacture laser device or a combined treatment and manufacture laser device. Typically, the same kinds of laser devices may be used for both manufacture of the cornea implant as well as resecting the patient cornea.

An ophthalmological laser device may in an embodiment include femtosecond laser source. A femtosecond laser source is particularly favorable in that it allows to implement the cornea cuts with high precision and reproducibility. Nano or picosecond laser in the UV range may be used in alternative embodiments.

A method for manufacturing a cornea implant in accordance with the present disclosure may include, prior to implementing donor cornea cuts, mounting the donor cornea on an artificial anterior chamber epithelium with the donor anterior cornea surface facing the manufacture laser device respectively ophthalmological laser device and applanating the donor cornea with a sterilized contact element.

Similarly, an ophthalmological surgical method not being part of this invention may include, prior to implementing patient cornea cuts, applanating the patient cornea with a sterilized contact element.

The donor access cut is generally a circumferential cut respectively extends circumferentially from a circumferential edge of the donor anterior lamellar cut as mentioned before. In an embodiment, the donor access cut is circumferentially closed. In such embodiment, the method for manufacturing the cornea implant may further include, subsequent to implementing the donor cornea cuts, lifting off or peeling off a donor cover element, the donor cover element being a portion of the donor cornea delimited by the donor anterior cornea surface, the donor anterior lamellar cut and the donor side cut. The donor cover element is the portion of the donor cornea anterior of the cornea implant, with the cornea implant and the donor cover element being separated by the donor anterior lamellar cut. Alternatively, however, the donor access cut is not circumferentially closed but extends only over a portion of the circumference of the donor anterior lamellar cut. In this case, the donor cover element remains connected to the further tissue of the donor cornea via a tissue bridge after implementing the donor cornea cuts, in particular the donor anterior lamellar cut and the donor access cut. **In** such case, the method may include flipping the donor cover element away along the tissue bridge, with the tissue bridge serving as hinge. The method may further include grasping the cornea implant, in particular using forceps, and removing it from the donor cornea.

The invention is defined by the appended claims.

### BRIEF DESCRIPTION OF THE FIGURES

The herein described invention will be more fully understood from the detailed description given herein below and the accompanying drawings which should not be considered limiting to the invention described in the appended claims. The drawings show:
- Fig. 1: an embodiment of an ophthalmological treatment and manufacture apparatus according to the present invention in a schematic sectional view;
- Fi. 2: a patient cornea with implemented patient cornea cuts according to an embodiment of the present invention in a schematic sectional side view;
- Fig. 3: the patient cornea with implemented patient cornea cuts corresponding to Figure 2 in a schematic top view;
- Fig. 4: a donor cornea with implemented donor cornea cuts according to an embodiment of the present invention in a schematic sectional side view;
- Fig. 5: the donor cornea with implemented donor cornea cuts corresponding to Figure 5 in a schematic top view.
- Fig. 6: a configuration for the insertion of a cornea implant into a patient cornea in accordance with the present invention in a schematic side view;
- Fig. 7: the positioning of a cornea implant in accordance with the present invention in a schematic top view.

### EXEMPLARY EMBODIMENTS

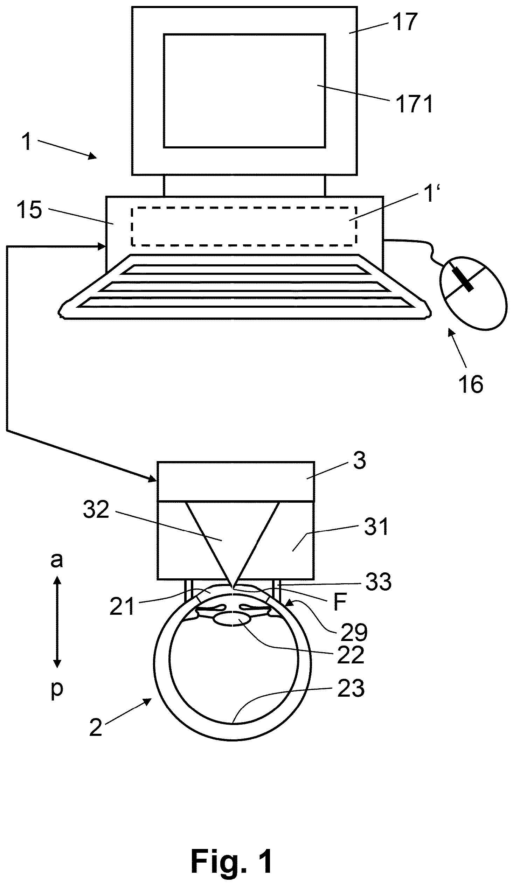

In the following, reference is first made to **Figure 1. Figure 1** shows an exemplary embodiment of an ophthalmological treatment and manufacture apparatus 1, 3 in accordance with the present disclosure and in interaction with a human eye (patient eye) 2 in a schematic view.

For the human eye 2, the cornea 21, the lens 22, the retina 23 and the sclera 29 are shown. Further, the limbus (not separately referenced) is present as transition area between the cornea 21 and the sclera 29.

In **Figure 1** as well as further figures, an anterior-pointing direction is indicated by "a" and a posterior-pointing direction is indicated by "p". The anterior-pointing direction a and the posterior-pointing direction p are defined in the same manner for the patient eye 2 as well as its parts, as well as a donor cornea and a cornea implant as illustrated in further figures below.

The ophthalmological treatment and manufacture apparatus comprises the computerized ophthalmological treatment and manufacture control device 1 (in the following generally referred to as control device) and the ophthalmological laser device 3 (in the following generally referred to as laser device) that is exemplarily based on a femtosecond laser source in operative coupling with a projection lens that is part of an application head (components as laser sources, scanners and beam delivery and shaping optics are not separately shown).

From the laser device 3 respective its projection lens, laser radiation is emitted and focused onto a point of the patient cornea 21 (indicated by radiation cone 32), or similarly, of a donor cornea. In a situation of use, laser device 3 is coupled to the patient eye 2 or a donor cornea via a patient interface 31 that is typically designed for one-way use and couples with the surgical laser device 3 respectively its application head via releasable mechanical coupling and for coupling with the eye 2 respective its sclera 29 and/or cornea 21 via a suction ring 33. Coupling is not mandatory if the laser can compensate for eye movement or is fast enough. The patient interface 31 may be designed to deform the eye surface to a desired shape for the surgical procedure, e. g. planar, spherical or aspherical shape with an applanation-type coupling and a transparent contact element. Alternatively, the patient interface 31 may be designed to couple with the patient eye 2, in particular the patient cornea 21, or a donor cornea, via a contact liquid, e. g. saline solution, substantially without causing deformation. An exemplary patient interface is disclosed in the EP2853247.

The control device 1 is exemplarily shown as being based on a general-purpose computing device 15, such as a PC or workstation, in operative coupling with a user interface that includes an input unit 16 (shown with a keyboard and a mouse for exemplary purposed) and an output unit 17 (exemplarily shown as computer monitor with an image area 171. Other devices for the input unit 16 and/or the output unit 17 may be used as well. For example, the input unit 16, may include a track ball and/or a touch pad. The output unit 17 may, for example, be or include a beamer or glasses with integrated display, to be worn by an operator.

The control device 1 includes a number of functional modules 1', which include interface circuitry for coupling with the laser device 3 via an (typically electrical) operative coupling. The control device 1 further includes one or more processors, e. g. microprocessors and/or microcontrollers for controlling operation of the control device 1 and the ophthalmological treatment and manufacture apparatus as a whole in accordance with corresponding program code. The control device 1 further includes volatile and/or non-volatile memory that store program code and/or data.

In **Figure 1****,** the control device is 1 shown as a compact unit. The control device 1 may, however, also be distributed between a number of computing devices, each with particular functional units 1'. The computing devices may be coupled via data interfaces such as a LAN network or USB connection for data transfer and/or between which data may be transferred via physical media, such as CD-ROMS, DVDs, magnetic storage discs, or the like. Further, the control device 1 may be fully or partly integrated with the surgical laser device 3.

It is noted that rather than an ophthalmological treatment and manufacture apparatus, a dedicated and distinct ophthalmological treatment apparatus and ophthalmological manufacture apparatus may be used as explained in the general description to be used. While typically deviating regarding the program code and/or data, a distinct ophthalmological treatment apparatus and an ophthalmological manufacture apparatus may in each case generally be designed as depicted in **Figure 1****.**

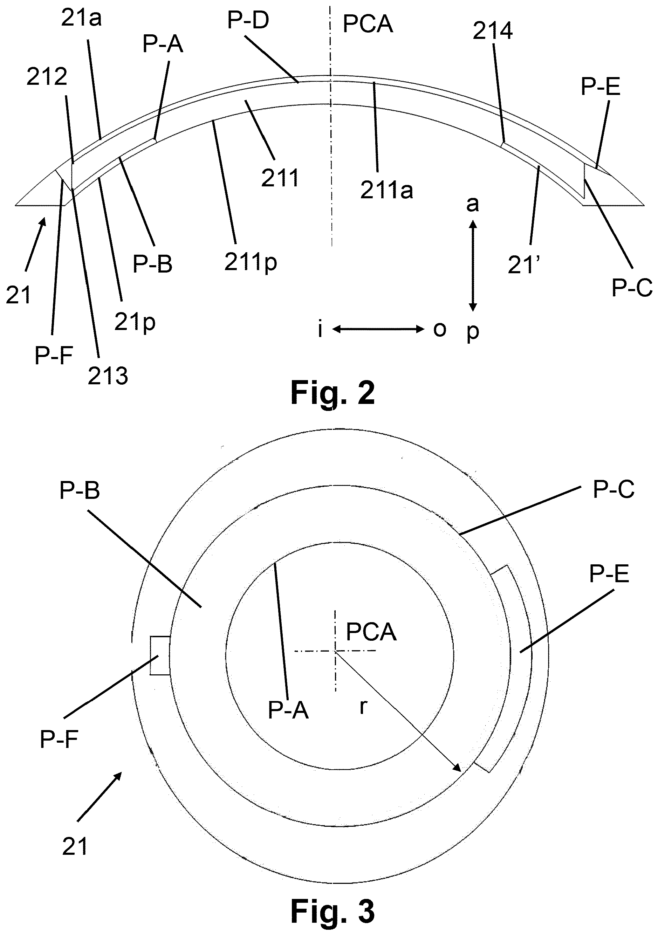

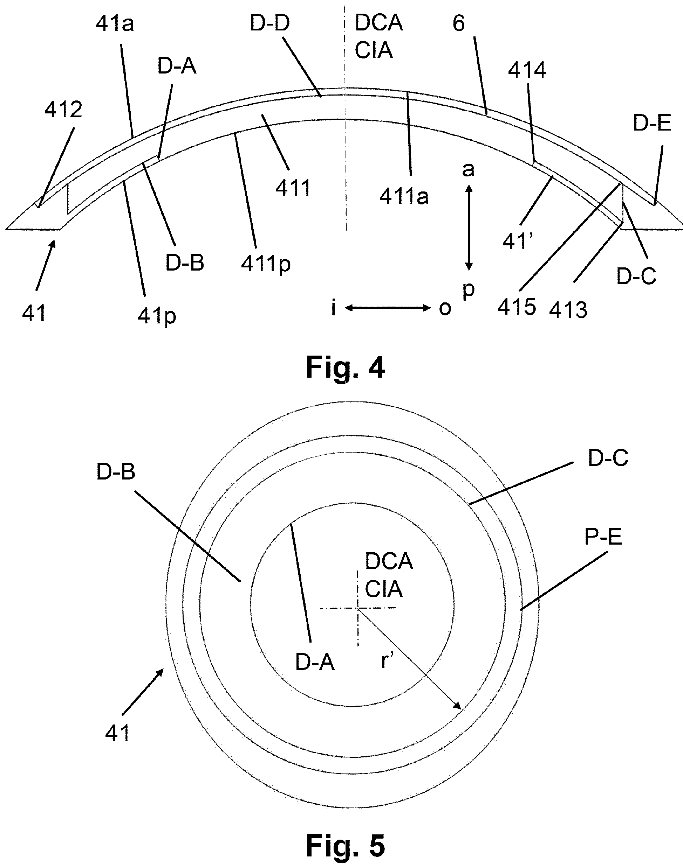

In the following, reference is additionally made to **Figures 2** and **Figure 3. Figure 2** shows a schematic sectional side view of the patient cornea 21 with the patient cornea element 211 and the patient cornea cuts respectively patient cornea cut pattern as described in the following. **Figure 3** shows a corresponding schematic top view of the patient cut pattern. The patient cornea axis is referenced with "PCA". It is noted that like in further figures as discussed below, in particular **Figure 4****,** hatchings are omitted for clarity reasons. Further it is noted that the top views of **Figure 3** and **Figure 5** are shown somewhat smaller as compared to the side sectional views of **Figure 2** and **Figure 4****.** It is noted that the figures generally show the cornea and cornea cuts in their natural shape, i.e. without applanating, for clarity reasons. Also references to the geometry of particular elements or cuts are made with reference to this configuration, if not stated differently.

An anterior-pointing respectively posterior-pointing direction is referenced with "a" respectively "p". An inwards-pointing direction (towards a center of the patient cornea 21) is referenced with "i" and an outwards-pointing direction (towards the periphery of the patient cornea 21) is referenced with "o". The same notation is used in further figures below for the donor cornea and the cornea implant in an analogue manner. It is noted that the inwards-pointing direction I and the outwards-pointing direction o are radial respectively lateral directions. It is noted in this context that the patient cornea, the donor cornea as well as the cornea implant are here generally assumed as rotationally symmetric, which is typically approximately but not necessarily strictly the case in practice.

The patient anterior lamellar cut P-D extends in the patient cornea 21 and approximately equidistant to the patient anterior cornea surface 21 and has for example a diameter of about 10mm. The patient anterior lamellar cut P-D lies fully within the patient cornea 21 between the patient anterior cornea surface 21a and the patient posterior cornea surface 21p. The patient anterior lamellar cut P-D extends in a substantially equidistant manner to the patient anterior cornea surface 21a as well as in substantially equidistant manner to the patient posterior cornea surface 21p. Typically, the patient anterior lamellar cut P-D is further substantially spherical and centered with respect to the patient cornea axis PCA. The patient anterior lamellar cut P-D is spaced apart from the patient anterior cornea surface 21a by about 1 00micrometer or somewhat more in a typical embodiment. The same applies for the distance between the donor anterior lamellar cut D-D and the donor cornea surface as discussed further below. Generally, a small distance between the patient respectively donor anterior cornea surface and the patient respectively donor anterior side cut is preferably, without, while ensuring that a sufficient amount of tissue remains, in particular at the patient cornea, to ensure stability respectively avoid damage of the cornea tissue anterior of the patient cornea element.

The patient side cut P-C is a circumferential cut and extends from the circumferential edge 212 of the patient anterior lamellar cut towards P-D towards the patient posterior cornea surface 21p in a circumferentially closed manner. In the shown embodiment, the patient side cut P-C is for example an inverted 150 degrees cut in the applanated state and may run substantially parallel to the patient cornea axis PCA, which however is not essential. In alternative embodiments, the patient side cut may be angled respectively oblique to the patient cornea axis PCA. In the shown design, the patient side cute P-C does not extend full to the patient posterior cornea surface 21p, bud ends within the patient cornea, spaced apart from the patient posterior cornea surface 21p for interlocking purposes as explained further below. It is noted that the circumferential edge 212 of the patient anterior lamellar cut is at the same time an anterior edge of the patient side cut P-C.

In the shown embodiment, a patient posterior lamellar cut P-B runs from the posterior edge 213 of the patient side cut P-C inwards. The patient posterior lamellar cut P-B extends in a circumferentially closed manner inward. In the shown embodiment, the patient posterior lamellar cut P-B extends with constant distance to the patient anterior lamellar cut P-D in a coaxial respectively centered manner and is preferably spherical. It is noted that the posterior edge 213 of the patient side cut P-C is at the same time a circumferential outer edge of the patient posterior lamellar cut P-B. Further, a patient auxiliary side cut P-A runs from a circumferential inner edge 214 of the patient posterior lamellar cut P-B to the patient posterior cornea surface 21p and extends to the posterior cornea surface 21p.

The patient anterior lamellar cut P-D, the patient posterior lamellar cut P-B and the patient posterior cornea surface 21p delimit, in combination, a patient cornea element 211 in axial respectively anterior-posterior-direction. Further, the patient side cut P-C and patient auxiliary side cut P-A delimit the patient cornea element 211 in the radial direction. Via patient cornea cuts P-A, P-B, P-C, P_D, the patient cornea element 211 is separated from the further tissue of the patient cornea 21. The patient cornea element 211 is formed by respectively comprises the generally pathological respectively defective portion of the patient cornea that is resected and subsequently replaced by a cornea implant. The patient anterior cornea element surface is defined by the patient anterior lamellar cut P-D, while the patient posterior cornea element surface 211b is defined by the patient posterior cornea surface 21p in a central part (in radial direction from the patient cornea axis PCA to the patient auxiliary side cut P-A), and by the patient posterior lamellar cut P-B in a peripheral part (in radial direction outwards from the patient auxiliary side cut P-A).

Via the patient posterior lamellar cut P-B and the patient auxiliary side cut P-A, an annular step is formed in the patient posterior cornea element surface 211p, respectively an annular recess is formed at the posterior side of the patient cornea element 211. The portion of tissue that is separated from the patient cornea element 211 by the patient posterior lamellar cut P-B and the patient auxiliary side cut P-A is a patient cornea tissue ring 21' and is complementary to the recess in the patient cornea element 211.

In addition to the patient cornea cuts P-A, P-B, P-C, P-D which define, in combination the shape of the patient cornea element 211, the patient cornea cut pattern includes two additional patient cornea cuts that serve the surgical procedure, namely a patient access cut P-E and an auxiliary patient access cut P-F. A patient access cut P-E extends in the patient cornea 21 from the patient side cut P-C to a peripheral region of the patient anterior cornea surface 21a. In the shown design, the patient access cut P-E meets the patient side cut P-C at its anterior edge 212. It could, however, also meet the patient side cut P-C somewhat further posterior in direction towards its posterior edge 213. Favorably, the patient access cut P-E meets the patient anterior cornea surface in an acute respectively shallow angle. The patient access cut P-E may be implanted starting at the periphery anterior cornea surface 21a and ending at the patient side cut P-E.

In the shown embodiment, the patient access cute P-E meets the patient anterior cornea surface 21a as a circular arc and generally concentrically to the patient cornea axis PCA: This, however, is not essential. In alternative embodiments, the patient access cut P-E may meet patient anterior cornea surface 21a in a straight line or as outwards-curved arc, i.e. away from the patient cornea axis P-A.

As best visible in **Figure 3****,** the patient access cut is a sectoral cutthat is not circumferentially thoroughgoing but extends over an arc length in a typical range of about 4mm to 5mm.

The patient access cut P-E provides a tunnel-shaped access channel for removing the patient cornea element 211 as well as inserting and positioning the cornea implant. The patient access cut is favorably shaped to allow the insertion of an insertion spatula.

In the shown embodiment, an auxiliary patient access cut P-F is provided diametrically opposed to the patient access cut P-E. The auxiliary patient access cut P-F extends from the patient side cut P-C to the peripheral region of the patient anterior cornea surface 21a. In the shown design, the auxiliary patient access cut meets the patient side cut P-C at its posterior edge 213. It could, however, also meet the patient side cut P-C somewhat further anterior in direction towards its anterior edge 212. The auxiliary patient access cut P-F provides a tunnel-shaped auxiliary access channel for positioning the cornea implant with an auxiliary forceps that is introduced via the auxiliary patient access cut P-F.

While the patient cornea cuts may generally be implemented respectively executed in different orders, an advantageous order that is particularly favorable is P-A, P-B, P-C, P-D, P-E, P-F.

After removing the patient cornea element 211, a patient cornea cavity of generally same shape and dimension will be present in the patient cornea.

In the following, reference is additionally made to **Figures 4** and **Figure 5. Figure 4** shows a schematic sectional side view of the donor cornea 41 from which the cornea implant 411 is manufactured respectively produced, as well as the donor cornea cuts respectively the donor cornea cut pattern. **Figure 5** shows a corresponding schematic top view of the donor cut pattern. The donor cornea axis is referenced with "DCA", generally corresponding to the cornea implant axis CIA which is referenced as CIA. Since a number of donor cornea cuts corresponds to patient cornea cuts as discussed before, the following description generally focusses on the differences and particular aspects of the manufacture of cornea implant 411.

The donor side cut D-C, the donor posterior lamellar cut D-B and the donor auxiliary side cut D-A generally correspond to the patient side cut P-C, the patient posterior lamellar cut P-B, and the patient auxiliary side cut P-A. The donor auxiliary side cut D-A, however, may have in a particular implementation have a somewhat smaller diameter in a viewing direction along the donor cornea axis DCA as compared to the patient auxiliary side cut P-A in a viewing direction along the patient cornea axis PCA, respectively the donor auxiliary side cut D-A may be somewhat closer to the donor cornea axis DCA than the patient auxiliary side cut P-A is to the patient cornea axis PCA. The distance between the patient anterior lamellar cut P-D to the patient posterior lamellar cut B-B is generally identical to the distance between the donor anterior lamellar cut D-D and the donor posterior lamellar cut D-B.

Further, the radial extension of the donor posterior lamellar cut D-B may in an embodiment be somewhat larger as compared to the patient posterior lamellar cut P-B. In this way, the radius r' (see Figure 5) of the cornea implant 411 is slightly larger as compared to the radius r of the patient cornea element 211, respectively the lateral dimension of the cornea implant 411 is slightly larger respectively has a slight oversize as compared to the patient cornea element 211. In this way, it is ensured that the cornea implant sits snugly in the patient cornea cavity after insertion.

Other embodiments, however, may be used as well. In particular, the patient cornea cuts and the corresponding donor cornea cuts may correspond to each other as closely as possible.

Further, the lateral extension of the donor anterior lamellar cut D-D may in an embodiment be wider respectively the circumferential edge 412 of the donor anterior lamellar cut D-E as compared to the patient anterior lamellar cut P-D. As a consequence, the donor side cut D-C does not meet with the donor anterior lamellar cut D-D at its circumferential edge 412. Instead, the donor side cut D-C is displaced inwards and a junction of its anterior edge 415 with the donor anterior lamellar cut is displaced inwards respectively within the area as delimited by the circumferential edge 412 of the donor anterior lamellar cut D-D. Further, the donor access cut D-E is a circumferential cut that extends from the circumferential edge 412 to the donor anterior cornea surface 41a.

As a consequence of the here-described cut arrangement a donor cover element 6 is created that laterally projects over the cornea implant 6 that is manufactured via the donor cornea cuts. The donor cover element 6 is generally separated from the further parts of the donor cornea 41 via the donor anterior lamellar cut D-D and the donor access cut D-E. In the shown embodiment, the donor access cut is circumferentially closed respectively thronging. Subsequent to implementing the donor cornea cuts, the donor cover element 6 can be simply peeled or lifted off, using a suited instrument such as forceps. Subsequently, the manufactured cornea implant 411 can be removed from the remaining tissue of the donor cornea 41. In an alternative embodiment, the donor side cut D-E is not circumferential through going, but a tissue bridge remains via which the donor cover element 6 may be flipped away in a hinge-like manner to provide access to the cornea implant 411.

When inserting and positioning the cornea implant 411 in the patient cornea cavity of the patient cornea 41, the peripheral part of the cornea implant posterior surface 411p will abut the anterior surface of the patient cornea tissue ring 21' such that the cornea implant 411 rests on the patient cornea tissue ring 21'. In a variant, the cornea tissue ring 21' does not extend transverse to the patient cornea axis PCA respectively cornea implant axis CIA, but extend oblique, in particular in anterior direction. The circumferential recess of the cornea implant 411 is designed accordingly in this case. For the donor the same applies in an analogue manner.

While the donor cornea cuts may generally be implanted in different orders, an advantageous order that is particularly favorable is D-A, D-B, D-C, D-D, D-E.

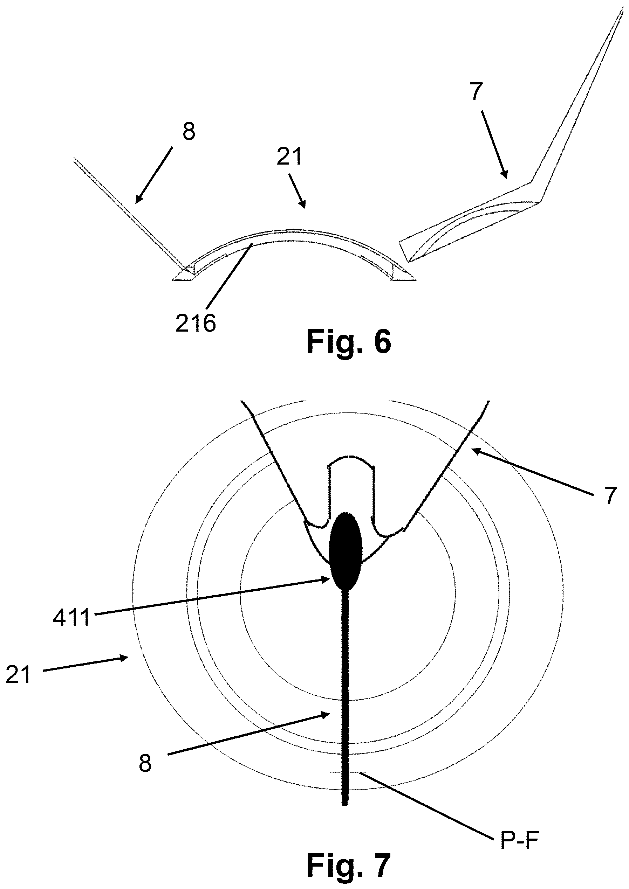

In the following, reference is additionally made to **Figure 6** and **Figure 7****,** schematically illustrating the insertion and placing of the cornea implant 411 in the patient cornea 21. **Figure 6** illustrated the situation prior the actual insertion if the cornea implant 411. An insertion spatula 7 with the cornea implant 411 (not visible in **Figure 6**) is positioned to allow introduction via the patient access cut P-E (not visible in **Figures 6, 7**) into the patient cornea cavity 216 which has been created before by resecting and removing the patient cornea tissue element 211 as mentioned. Further, auxiliary forceps are positioned and introduced into the patient cornea cavity 416 via the auxiliary patient access cut P-F.

**Figure 7** illustrates the positioning of the cornea implant 411 in a schematic top view. By grasping the cornea implant 411 with auxiliary forceps 8, the cornea implant 8 can maneuvered, in particular pulled, into the correct position within the patient cornea cavity 216. In the schematic illustration of **Figure 7****,** the cornea implant 411 is directly grasped in a peripheral region by auxiliary forceps 8. It may, however, also be manipulated differently, for example via a length of suture which is fixed to the cornea implant 411 in its peripheral region.

### REFERENCE SIGNS

- 1: ophthalmological treatment and manufacture apparatus control device / control device
- 1': functional unit
- 15: general-purpose computing device
- 16: input unit
- 17: output unit
- 171: image area
- 2: patient eye
- 21: patient cornea
- 21': patient cornea tissue ring
- 21a: patient anterior cornea surface
- 21p: patient posterior cornea surface
- 211: patient cornea element
- 211a: patient anterior cornea element surface
- 211p: patient posterior cornea element surface
- 212: circumferential edge of patient anterior lamellar cut / anterior edge of patient side cut
- 213: circumferential outer edge of patient posterior lamellar cut / posterior edge of patient side cut
- 214: circumferential inner edge of patient posterior lamellar cut / anterior edge of patient auxiliary side cut
- 216: patient cornea cavity
- 22: lens
- 23: retina
- 29: sclera
- 3: ophthalmological laser device / laser device
- 31: patient interface
- 32: radiation cone
- 33: suction ring
- 41: donor cornea
- 41': donor cornea tissue ring
- 41a: donor anterior cornea surface
- 41p: donor posterior cornea surface
- 411: cornea implant
- 411a: cornea implant anterior surface
- 411p: cornea implant posterior surface
- 412: circumferential edge of donor anterior lamella cut; posterior edge of patient access cut
- 413: circumferential outer edge of donor posterior lamellar cut / posterior edge of donor side cut
- 414: circumferential inner edge of donor posterior lamellar cut / anterior edge of auxiliary side cut
- 415: anterior edge of donor side cut
- 5: cornea implant
- 5a: cornea implant anterior surface
- 5p: cornea implant posterior surface
- 6: donor cover element
- 7: insertion spatula
- 8: auxiliary forceps
- a: anterior-point direction
- p: posterior-pointing direction
- i: inwards-pointing direction
- o: outwards-pointing direction
- r: radius of patient cornea element
- r': radius of cornea implant
- CIA: corneal implant axis
- PCA: patient cornea axis
- DCA: donor cornea axis
- P-A: patient auxiliary side cut
- P-B: patient posterior lamellar cut
- P-C: patient side cut
- P-D: patient anterior lamellar cut
- P-E: patient access cut
- P-F: auxiliary patient access cut
- D-A: donor auxiliary side cut.
- D-B: donor posterior lamellar cut
- D-C: donor side cut
- D-D: donor anterior lamellar cut
- D-E: donor access cut

## Claims

1. Ophthalmological treatment apparatus (1, 2) for resecting a patient cornea element from a patient cornea (21) in posterior lamellar keratoplasty, the ophthalmological treatment apparatus (1, 2) including:
a) a treatment laser device (3) for implementing cornea cuts;
b) a computerized treatment control device (1) in operative coupling with the treatment laser device (3), the treatment control device (1) being designed to control the treatment laser device (3) to implement a number of patient cornea cuts, the patient cornea cuts forming, in combination, a patient cornea cut pattern with a geometry of the patient cornea element (211) being defined by the patient cornea cut pattern,
the number of patient cornea cuts including:
- a patient anterior lamellar cut (P-D), the patient anterior lamellar cut (P-D) extending in the patient cornea (21) at a patient anterior lamellar cut distance from a patient posterior cornea surface (41p) of the patien211t cornea (21), the patient anterior lamellar cut (P-D) defining a patient anterior cornea element surface (211a),
- a patient side cut (P-C), the patient side cut (P-C) extending in the patient cornea (21) from a periphery, in particular a circumferential edge, of the patient anterior lamellar cut (P-D) towards the patient posterior cornea surface (41p) in a circumferentially closed manner, the patient side cut (P-C) defining, at least in part, a circumferential patient cornea element surface, wherein the patient side cut (P-C) does not extend to the patient posterior cornea surface (41p),
- a patient access cut (P-E), the patient access cut (P-E) extending in the patient cornea (21) from the patient side cut (P-C) to a peripheral region of the patient anterior cornea surface (21a), the patient access cut (P-E) forming an access passage in the patient cornea (21),
- a patient posterior lamellar cut (P-B), the patient posterior lamellar cut (P-B) extending circumferentially in the patient cornea (21) from a posterior end, in particular a posterior edge (213), of the patient side cut (P-C) inwards with respect to the patient cornea (21), in particular with a constant distance to the patient anterior lamellar cut (P-D),
- a patient auxiliary side cut (P-A), the patient auxiliary side cut (P-A) extending from an inner periphery, in particular an inner edge (214), of the patient posterior lamellar cut (P-B) to the patient posterior cornea surface (41p),
the patient cornea cut pattern further defining a patient cornea interlocking structure, the patient cornea interlocking structure being configured to interlock the patient cornea (21) with a cornea implant (411) after replacing the patient cornea element (211) by the cornea implant (411).

2. Ophthalmological treatment apparatus (1, 2) according to claim 1, wherein the treatment control device (1) is configured to control the treatment laser device (3) to implement the patient anterior lamellar cut (P-D) subsequent to the patient side cut (P-C) and the patient access cut (P-E) subsequent to the patient anterior lamellar cut (P-D).

3. Ophthalmological treatment apparatus (1, 2) according to one of claims 1 to 2, wherein the treatment control device (1) is configured to control the treatment laser device (3) to implement the patient posterior lamellar cut (P-B) subsequent to the patient auxiliary side cut (P-A) and prior to the patient side cut (P-C).

4. Ophthalmological treatment apparatus (1, 2) according to one of claims 1 to 3, wherein the patient cornea interlocking structure is defined, at least in part, by a patient posterior cornea element surface (211p).

5. Ophthalmological treatment apparatus (1, 2) according to one of claims 1 to 4, wherein the patient cornea element (211) includes a circumferential step in the patient posterior cornea element surface (211p), in particular in a peripheral region of the patient posterior cornea element surface (211p).

6. Ophthalmological treatment apparatus (1, 2) according to one of claims 1 to 5, wherein the patient access cut (P-E) meets the patient anterior cornea surface (21a) in an acute angle.

7. Ophthalmological treatment apparatus (1, 2) according to any one of claims 1 to 6, wherein the patient cornea cut pattern includes an auxiliary patient access cut (P-F), the auxiliary patient access cut (P-F) extending in the patient cornea (21) from the patient side cut (P-C) to the peripheral region of the patient anterior cornea surface (21a), wherein the auxiliary patient access cut (P-F) is arranged with respect to a circumference of the patient side cut (P-C) opposite to the patient access cut (P-E), the auxiliary patient access cut (P-F) forming an auxiliary access passage in the patient cornea (21).

8. Ophthalmological treatment apparatus (1, 2) according to claim 7, wherein the patient auxiliary access cut (P-E) meets the patient anterior cornea surface (21a) in a perpendicular or substantially perpendicular manner.

9. Ophthalmological treatment apparatus (1,2) according to one of claim 1 to claim 8, wherein the treatment control device (1) is configured to implement the patient cornea cuts that define the geometry of the patient cornea element (211) in sequence one after the other.

10. Ophthalmological manufacture apparatus (1, 2) for manufacturing from a donor cornea (41) a cornea implant (411) suited for use in in posterior lamellar keratoplasty, the ophthalmological manufacture apparatus (1, 2) including:
a) a manufacture laser device (3) for implementing cornea cuts;
b) a computerized manufacture control device (1) in operative coupling with the manufacture apparatus (3), the manufacture control device (1) being designed to control the manufacture laser device (3) to implement a number of donor cornea cuts, the donor cornea cuts forming, in combination, a donor cornea cut pattern with a geometry of the cornea implant (411) being defined by the donor cornea cut pattern,
the number of donor cornea cuts including:
- a donor anterior lamellar cut (D-D), the donor anterior lamellar cut (D-D) extending in the donor cornea (41) at a donor anterior lamellar cut distance from a donor posterior cornea surface (41p), the donor anterior lamellar cut (D-D) defining a cornea implant anterior surface (411a),
- a donor side cut (D-C), the donor side cut (D-C) extending in the donor cornea (41) from the donor anterior lamellar cut (D-D) towards the donor posterior cornea surface (41p) in a circumferentially closed manner, wherein a junction of the donor side cut (D-C) and the donor anterior lamellar cut (D-D) is displaced with respect to a circumferential edge (412) of the donor anterior lamellar cut (D-D), the donor side cut (D-C) defining, at least in part, a circumferential cornea implant surface, wherein the donor side cut (D-C) does not extend to the donor posterior cornea surface (41d),
- a donor access cut (D-E), the donor access cut (D-E) extending from a periphery, in particular a circumferential edge (412), of the donor anterior lamellar cut (D-D) to a donor anterior cornea surface (41a),
- a donor posterior lamellar cut (D-B), the donor posterior lamellar cut (D-B) extending in the donor cornea from a posterior edge (413), of the donor side cut (D-C) inwards with respect to the donor cornea (41), in particular with a constant distance to the donor anterior lamellar cut (D-D),
- a donor auxiliary side cut (D-A), the donor auxiliary side cut (D-A) extending from an inner edge (414), of the donor posterior lamellar cut (D-B) to the donor posterior cornea surface (41d),
the donor cornea cut pattern further defining a cornea implant interlocking structure, the cornea implant interlocking structure being configured to interlock a patient cornea (21) with the cornea implant (411) after replacing the patient cornea element (211) by the cornea implant (411).

11. Method for manufacturing from a donor cornea (41) a cornea implant (411) suited for use in posterior lamellar keratoplasty, in particular using an ophthalmological manufacture apparatus (1, 2) according to claim 10, the method not being practised on the living human or animal body,
wherein the method includes implementing by means of a laser device, in particular an ophthalmological laser device (3), a number of donor cornea cuts, the donor cornea cuts forming, in combination, a donor cornea cut pattern with a geometry of the cornea implant (411) being defined by the donor cornea cut pattern,
the number of donor cornea cuts including:
- a donor anterior lamellar cut (D-D), the donor anterior lamellar cut (D-D) extending in the donor cornea (41) at a donor anterior lamellar cut distance from the donor posterior cornea surface (41p), the donor anterior lamellar cut (D-D) defining a cornea implant anterior surface (411a);
- a donor side cut (D-C), the donor side cut (D-C) extending in the donor cornea (41) from the donor anterior lamellar cut (D-D) towards the donor posterior cornea surface (41p) in a circumferentially closed manner, wherein a junction of the donor side cut (D-C) and the donor anterior lamellar cut (D-D) is displaced with respect to a circumference edge (412) of the donor anterior lamellar cut (D-D), the donor side cut (D-C) defining, at least in part, a circumferential cornea implant surface, wherein the donor side cut (D-C) does not extend to the donor posterior cornea surface (41d),
- a donor access cut (D-E), the donor access cut (D-E) extending from a periphery, in particular a circumferential edge, of the donor anterior lamellar cut (D-D) to a donor anterior cornea surface (41a),
- a donor posterior lamellar cut (D-B), the donor posterior lamellar cut (D-B) extending in a donor cornea from a posterior edge (413), of the donor side cut (D-C) inwards with respect to the donor cornea (41), in particular with a constant distance to the donor anterior lamellar cut (D-D),
- a donor auxiliary side cut (D-A), the donor auxiliary side cut (D-A) extending from an inner edge (414), of the donor posterior lamellar cut (D-B) to the donor posterior cornea surface (41d),
the donor cornea cut pattern further defining a cornea implant interlocking structure, the cornea implant interlocking structure being configured to interlock a patient cornea (21) with the cornea implant (411) after replacing the patient cornea element (211) by the cornea implant (411).

## Patentansprüche

1. Ophthalmologische Behandlungsvorrichtung (1, 2) zum Resezieren eines Patientenkorneaelements aus einer Patientenkornea (21) bei einer posterioren lamellären Keratoplastik, wobei die ophthalmologische Behandlungsvorrichtung (1, 2) umfasst:
a) eine Behandlungslaservorrichtung (3) zum Ausführen von Korneaschnitten;
b) eine rechnergestützte Behandlungssteuervorrichtung (1) in operativer Kopplung mit der Behandlungslaservorrichtung (3), wobei die Behandlungsteuervorrichtung (1) ausgebildet ist, die Behandlungslaservorrichtung (3) zu steuern, um eine Anzahl an Patientenkorneaschnitten durchzuführen, wobei die Patientenkorneaschnitte in Kombination ein Patientenschnittmuster bilden, mit einer Geometrie des Patientenkorneaelements (211), die durch das Patientenschnittmuster definiert ist,
wobei die Anzahl der Patientenkorneaschnitte umfasst:
- einen anterioren lamellären Patientenschnitt (P-D), wobei sich der anteriore lamelläre Patientenschnitt (P-D) in der Patientenkornea (21) in einem anterioren lamellären Patientenschnittabstand von einer posterioren Patientenkorneaoberfläche (41p) der Patientenkornea (21) erstreckt, wobei der anteriore lamelläre Patientenschnitt (P-D) eine anteriore Patientenkorneaelementoberfläche (211a) definiert,
- einen Patientenseitenschnitt (P-C), wobei sich der Patientenseitenschnitt (P-C) in der Patientenkornea (21) von einer Peripherie, insbesondere einer umlaufende Kante, des anterioren lamellären Patientenschnitts (P-D) in Richtung der posterioren Patientenkorneaoberfläche (41p) umlaufend geschlossen erstreckt, wobei der Patientenseitenschnitt (P-C) mindestens teilweise eine umlaufende Patientenkorneaelementoberfläche definiert, wobei sich der Patientenseitenschnitt (P-C) nicht bis zur posterioren Patientenkorneaelementoberfläche (41p) erstreckt,
- einen Patientenzugangsschnitt (P-E), wobei sich der Patientenzugangsschnitt (P-E) in der Patientenkornea (21) vom Patientenseitenschnitt (P-C) zu einem peripheren Bereich der anterioren Patientenkorneaoberfläche (21a) erstreckt, wobei der Patientenzugangsschnitt (P-E) einen Zugangskanal in der Patientenkornea (21) bildet,
- einen posterioren lamellären Patientenschnitt (P-B), wobei sich der posteriore lamelläre Patientenschnitt (P-B) in der Patientenkornea (21) umlaufend von einem posterioren Ende, insbesondere einer posterioren Kante (213), des Patientenseitenschnitts (P-C) nach innen in Bezug auf die Patientenkornea (21) erstreckt, insbesondere mit einem konstanten Abstand zum anterioren lamellären Patientenschnitt (P-D),
- einen Patientenhilfsseitenschnitt (P-A), wobei sich der Patientenhilfsseitenschnitt (P-A) von einer inneren Peripherie, insbesondere einer inneren Kante (214), des posterioren lamellären Patientenschnitts (P-B) zur posterioren Patientenorneaoberfläche (41p) erstreckt,
wobei das Patientkorneaschnittmuster ferner eine Patientenkorneaineinandergreifstruktur definiert, wobei die Patientenkorneaineinandergreifstruktur eingerichtet ist, die Patientenkornea (21) nach Ersetzen des Patientenkorneaelements (211) durch das Korneaimplantat (411) mit dem Korneaimplantat (411) ineinanderzugreifen.

2. Ophthalmologische Behandlungsvorrichtung (1, 2) gemäß Anspruch 1, wobei die Behandlungssteuervorrichtung (1) eingerichtet ist, die Behandlungslaservorrichtung (3) so zu steuern, dass sie den anterioren lamellären Patientenschnitt (P-D) nach dem Patientenseitenschnitt (P-C) und den Patientenzugangsschnitt (P-E) nach dem anterioren lamellären Patientenschnitt (P-D) durchführt.

3. Ophthalmologische Behandlungsvorrichtung (1, 2) gemäß einem der Ansprüche 1 bis 2, wobei die Behandlungsteuervorrichtung (1) eingerichtet ist, die Behandlungslaservorrichtung (3) so zu steuern, dass sie den posterioren lamellären Patientenschnitt (P-B) nach dem Patientenhilfsseitenschnitt (P-A) und vor dem Patientenseitenschnitt (P-C) des Patienten durchführt.

4. Ophthalmologische Behandlungsvorrichtung (1, 2) gemäß einem der Ansprüche 1 bis 3, wobei die Patientenkorneaineinandergreifstruktur zumindest teilweise durch eine posteriore Patientenkorneaelementoberfläche (211p) definiert ist.

5. Ophthalmologische Behandlungsvorrichtung (1, 2) gemäß einem der Ansprüche 1 bis 4, wobei das Patientenkorneaelement (211) eine Umlaufstufe in der posterioren Patientenkorneaelementoberfläche (211p) umfasst, insbesondere im peripheren Bereich der posterioren Patientenkorneaelementoberfläche (211p).

6. Ophthalmologische Behandlungsvorrichtung (1, 2) gemäß einem der Ansprüche 1 bis 5, wobei der Patientenzugangsschnitt (P-E) in einem spitzen Winkel auf die anteriore Patientenkorneaoberfläche (21a) trifft.

7. Ophthalmologische Behandlungsvorrichtung (1, 2) gemäß einem der Ansprüche 1 bis 6, wobei das Patientenkorneaschnittmuster einen zusätzlichen Patientenzugangsschnitt (P-F) umfasst, wobei sich der zusätzliche Patientenzugangsschnitt (P-F) in der Patientenkornea (21) vom Patientenkorneaschnitt (P-C) zum peripheren Bereich der anterioren Patientenkorneaoberfläche (21a) erstreckt, wobei der zusätzliche Patientenzugangsschnitt (P-F) in Bezug auf einen Umfang des Patientenseitenschnitts (P-C) gegenüber dem Patientenzugangsschnitt (P-E) angeordnet ist, wobei der zusätzliche Patientenzugangsschnitt (P-F) einen zusätzlichen Zugangskanal in der Patientenkornea (21) bildet.

8. Ophthalmologische Behandlungsvorrichtung (1, 2) gemäß Anspruch 7, wobei der zusätzliche Patientenzugangsschnitt (P-E) senkrecht oder im Wesentlichen senkrecht auf die anteriore Patientenkorneaoberfläche (21a) trifft.

9. Ophthalmologische Behandlungsvorrichtung (1, 2) gemäß einem der Ansprüche 1 bis 8, wobei die Behandlungssteuervorrichtung (1) eingerichtet ist, die Patientenkorneaschnitte, die die Geometrie des Patientenkorneaelements (211) definieren, sequentiell nacheinander auszuführen.

10. Ophthalmologische Herstellungsvorrichtung (1, 2) zur Herstellung eines Korneaimplantats (411) aus einer Spenderkornea (41), das zur Verwendung in der posterioren lamellären Keratoplastik geeignet ist, wobei die ophthalmologische Herstellungsvorrichtung (1, 2) umfasst:
a) eine Herstellungslaservorrichtung (3) zum Durchführen von Korneaschnitten;
b) eine rechnergestützte Herstellungssteuervorrichtung (1), die mit der Herstellungssvorrichtung (3) in betriebsfähige Kopplung ist, wobei die Herstellungssteuervorrichtung (1) ausgebildet ist, die Herstellungslaservorrichtung (3) zu steuern, um eine Anzahl an Spenderkorneaschnitten durchzuführen, wobei die Spenderkorneaschnitte in Kombination ein Spenderkorneaschnittmuster bilden, mit einer Geometrie des Korneaimplantats (411), die durch das Spenderkorneaschnittmuster definiert ist,
wobei die Anzahl an Spenderkorneaschnitten umfasst:
- einen anterioren lamellären Spenderschnitt (D-D), wobei sich der anteriore lamelläre Spenderschnitt (D-D) in der Spenderkornea (41) in einem anterioren lamellären Schnittabstand von einer posterioren Spenderkorneaoberfläche (41p) erstreckt, wobei der anteriore lamelläre Spenderschnitt (D-D) eine anteriore Korneaimplantatoberfläche (411a) definiert,
- einen Spenderseitenschnitt (D-C), wobei sich der Spenderseitenschnitt (D-C) in der Spenderkornea (41) vom anterioren lamellären Spenderschnitt (D-D) zur posterioren Spenderkorneaoberfläche (41p) umlaufend geschlossenen erstreckt, wobei eine Verbindungsstelle des Spenderseitenschnitts (D-C) und des anterioren lamellären Spenderschnitts (D-D) gegenüber einer umlaufenden Kante (412) des anterioren lamellären Spenderschnitts (D-D) versetzt ist, wobei der Spenderseitenschnitt (D-C) mindestens teilweise eine umlaufende Fläche des Korneaimplantats definiert, wobei sich der Spenderseitenschnitt (D-C) nicht bis zur posterioren Spenderkorneaoberfläche (41d) erstreckt,
- einen Spenderzugangsschnitt (D-E), wobei sich der Spenderzugangsschnitt (D-E) von einer Peripherie, insbesondere einer umlaufenden Kante (412), des anterioren lamellären Spenderschnitts (D-D) zu einer anterioren Spenderkorneaoberfläche (41a) erstreckt,
- einen posterioren lamellären Spenderschnitt (D-B), wobei sich der posteriore lamelläre Spenderschnitt (D-B) in der Spenderkornea von einer posterioren Kante (413) des Spenderseitenschnitts (D-C) nach innen in Bezug auf die Spenderkornea (41) erstreckt, insbesondere mit einem konstanten Abstand zum anterioren lamellären Spenderschnitt (D-D),
- einen Spenderhilfsseitenschnitt (D-A), wobei sich der Spenderhilfsseitenschnitt (D-A) von einer inneren Kante (414) des posterioren lamellären Spenderschnitts (D-B) zur posterioren Spenderkorneaoberfläche (41d) erstreckt,
wobei das Spenderkorneaschnittmuster ferner eine Korneaimplantatineinandergreifstruktur definiert, wobei die Korneaimplantatineinandergreifstruktur eingerichtet ist, eine Patientenkornea (21) nach Ersetzen des Patientenkorneaelements (211) durch das Korneaimplantat (411) mit dem Korneaimplantat (411) ineinanderzugreifen.

11. Verfahren zur Herstellung eines Korneaimplantats (411) aus einer Spenderkornea (41), das zur Verwendung in der posterioren lamellären Keratoplastik geeignet ist, insbesondere unter Verwendung einer ophthalmologischen Herstellungsvorrichtung (1, 2) gemäß Anspruch 10, wobei das Verfahren nicht am lebenden menschlichen oder tierischen Körper durchgeführt wird,
wobei das Verfahren umfasst: Durchführen mittels einer Laservorrichtung, insbesondere einer ophthalmologischen Laservorrichtung (3) eine Anzahl an Spenderkorneakorneaschnitten, wobei die Spenderkorneakorneaschnitte zusammen ein Spenderkorneaschnittmuster bilden, mit einer Geometrie des Korneaimplantats (411), die durch das Spenderkorneaschnittmuster definiert ist,
wobei die Anzahl der Spenderkorneakorneaschnitte umfasst:
- einen anterioren lamellären Spenderschnitt (D-D), wobei sich der anteriore lamelläre Spenderschnitt (D-D) in der Spenderkornea (41) in einem anterioren lamellären Schnittabstand vom posterioren lamellären Spenderschnitt der Spenderkornea (41p) erstreckt und der anteriore lamelläre Spenderschnitt (D-D) eine anteriore Korneaimplantatoberfläche (411a) definiert;
- einen Spenderseitenschnitt (D-C), wobei sich der Spenderseitenschnitt (D-C) in der Spenderkornea (41) vom anterioren lamellären Spenderschnitt (D-D) zur posterioren Spenderkorneaoberfläche (41p) umlaufend geschlossenen erstreckt, wobei eine Verbindungsstelle des Spenderseitenschnitts (D-C) und des anterioren lamellären Spenderschnitts (D-D) gegenüber einer umlaufenden Kante (412) des anterioren lamellären Spenderschnitts (D-D) versetzt ist, wobei der Spenderseitenschnitt (D-C) mindestens teilweise eine Umlauffläche des Korneaimplantats definiert, wobei sich der Spenderseitenschnitt (D-C) nicht bis zur Spenderkorneaoberfläche (41d) erstreckt,
- einen Spenderzugangsschnitt (D-E), wobei sich der Spenderzugangsschnitt (D-E) von einer Peripherie, insbesondere einer umlaufenden Kante, des anterioren lamellären Spenderschnitts (D-D) zu einer anterioren Spenderkorneaoberfläche (41a) erstreckt,
- einen posterioren lamellären Spenderschnitt (D-B), wobei sich der posteriore lamelläre Spenderschnitt (D-B) in einer Spenderkornea von einer posterioren Kante (413) des Spenderseitenschnitts (D-C) nach innen in Bezug auf die Spenderkornea (41) erstreckt, insbesondere mit einem konstanten Abstand zum posterioren lamellären Spenderschnitt (D-D),
- einen Spenderhilfsseitenschnitt (D-A), wobei sich der Spenderhilfsseitenschnitt (D-A) von einer inneren Kante (414) des posterioren lamellären Spenderschnitts (D-B) zur posterioren Spenderkorneaoberfläche (41d) erstreckt,
wobei das Spenderkorneaschnittmuster ferner eine Korneaimplantatineinan-dergreifstruktur definiert, wobei die Korneaimplantatineinandergreifstruktur eingerichtet ist, eine Patientenkornea (21) nach Ersetzen des Patientenkor-neaelements (211) durch das Korneaimplantat (411) mit dem Korneaim-plantat (411) ineinanderzugreifen.

## Revendications

1. Appareil de traitement ophtalmologique (1, 2) destiné à réséquer un élément de cornée du patient à partir de une cornée du patient (21) dans une kératoplastie lamellaire postérieure, l'appareil de traitement ophtalmologique (1, 2) comprenant:
a) un dispositif laser de traitement (3) destiné à réaliser des incisions dans la cornée;
b) un dispositif de contrôle de traitement informatisé (1) en couplage opératoire avec le dispositif laser de traitement (3), le dispositif de contrôle de traitement (1) étant conçu pour commander le dispositif laser de traitement (3) afin de réaliser un certain nombre d'incisions dans la cornée du patient, les incisions dans la cornée du patient formant, en combinaison, un motif de coupe dans la cornée du patient, la géométrie de l'élément de cornée du patient (211) étant définie par le motif de coupe dans la cornée du patient,
le nombre d'incisions dans la cornée du patient incluant:
- une coupe lamellaire antérieure du patient (P-D), la coupe lamellaire antérieure du patient (P-D) étendue dans la cornée du patient (21) à une distance de coupe lamellaire antérieure du patient à partir d'une surface cornéenne postérieure du patient (41 p) de la cornée du patient (21), la coupe lamellaire antérieure du patient (P-D) définissant une surface antérieure de l'élément de cornée du patient (211a),
- une coupe latérale du patient (P-C), la coupe latérale du patient (P-C) étendue dans la cornée du patient (21) depuis une périphérie, en particulier un bord circonférentiel, de la coupe lamellaire antérieure du patient (P-D) vers la surface cornéenne postérieure du patient (41p) de manière circonférentiellement fermée, la coupe latérale du patient (P-C) définissant, au moins en partie, une surface circonférentielle de l'élément de cornée du patient, dans laquelle la coupe latérale du patient (P-C) ne s'étend pas jusqu'à la surface cornéenne postérieure du patient (41p),
- une coupe d'accès du patient (P-E), la coupe d'accès du patient (P-E) étendue dans la cornée (21) du patient depuis la coupe latérale du patient (P-C) jusqu'à une région périphérique de l'surface cornéenne antérieure du patient (21a), la coupe d'accès du patient (P-E) formant un passage d'accès dans la cornée (21) du patient,
- une coupe lamellaire postérieure du patient (P-B), la coupe lamellaire postérieure du patient (P-B) s'étendant circonférentiellement dans la cornée du patient (21) depuis une extrémité postérieure, en particulier un bord postérieur (213), de la coupe latérale du patient (P-C) vers l'intérieur par rapport à la cornée du patient (21), en particulier à une distance constante de la coupe lamellaire antérieure (P-D) du patient,
- une coupe latérale auxiliaire du patient (P-A) du patient, la coupe latérale auxiliaire (P-A) du patient s'étendant depuis une périphérie interne, en particulier un bord intérieur (214), de la coupe lamellaire postérieure (P-B) du patient jusqu'à la surface cornéenne postérieure (41p) du patient,
le motif de coupe de la cornée du patient définissant en outre une structure d'emboîtement de la cornée du patient, la structure d'emboîtement de la cornée du patient étant configurée pour interverrouiller la cornée du patient (21) avec un implant cornéen (411) après avoir remplacé l'élément de cornée du patient (211) par l'implant cornéen (411).

2. Appareil de traitement ophtalmologique (1, 2) selon la revendication 1, dans laquelle le dispositif de contrôle de traitement (1) est configuré pour commander le dispositif laser de traitement (3) afin de réaliser la coupe lamellaire antérieure du patient (P-D) après la coupe latérale du patient (P-C) et la coupe d'accès du patient (P-E) après la coupe lamellaire antérieure du patient (P-D).

3. Appareil de traitement ophtalmologique (1, 2) selon l'une des revendications 1 à 2, dans lequel le dispositif de contrôle de traitement (1) est configuré pour commander le dispositif laser de traitement (3) afin de réaliser la coupe lamellaire postérieure du patient (P-B) après la coupe latérale auxiliaire du patient (P-A) et avant la coupe latérale du patient (P-C).

4. Appareil de traitement ophtalmologique (1, 2) selon l'une des revendications 1 à 3, dans laquelle la structure d'emboîtement de la cornée du patient est définie, au moins en partie, par la surface postérieure de l'élément de cornée du patient (211p).

5. Appareil de traitement ophtalmologique (1, 2) selon l'une des revendications 1 à 4, dans laquelle l'élément de cornée du patient (211) comprend un gradin circonférentiel dans la surface postérieure de l'élément de cornée du patient (211p), en particulier dans une région périphérique de la surface cornéenne postérieure du patient (211p).

6. Appareil de traitement ophtalmologique (1, 2) selon l'une des revendications 1 à 5, dans lequel la coupe d'accès du patient (P-E) rencontre l'surface cornéenne antérieure du patient (21a) selon un angle aigu.

7. Appareil de traitement ophtalmologique (1, 2) selon l'une quelconque des revendications 1 à 6, dans lequel le motif de coupe de la cornée du patient comprend une coupe d'accès auxiliaire du patient (P-F), la coupe d'accès auxiliaire du patient (P-F) étendue dans la cornée du patient (21) depuis la coupe latérale du patient (P-C) jusqu'à la région périphérique de l'surface cornéenne antérieure du patient (21a), dans laquelle la coupe d'accès auxiliaire du patient (P-F) est arrangée par rapport à une circonférence de la coupe latérale du patient (P-C) opposée à la coupe d'accès (P-E), la coupe d'accès auxiliaire (P-F) formant un passage d'accès auxiliaire dans la cornée du patient (21).

8. Appareil de traitement ophtalmologique (1, 2) selon la revendication 7, dans laquelle la coupe d'accès auxiliaire du patient (P-E) rencontre l'urface cornéenne antérieure du patient (21a) de manière perpendiculaire ou substantiellement perpendiculaire.

9. Appareil de traitement ophtalmologique (1, 2) selon l'une des revendications 1 à 8, dans lequel le dispositif de contrôle de traitement (1) est configuré pour mettre en œuvre les incisions dans la cornée du patient qui définissent la géométrie de l'élément de cornée du patient (211) séquentiellement l'un après l'autre.

10. Appareil de fabrication ophtalmologique (1, 2) destiné à fabriquer, à partir d'une cornée de donneur (41), un implant cornéen (411) adapté à l'utilisation dans une kératoplastie lamellaire postérieure, l'appareil de fabrication ophtalmologique (1, 2) comprenant:
a) un dispositif laser de fabrication (3) destiné à réaliser des incisions dans la cornée;
b) un dispositif de contrôle de fabrication informatisé (1) couplé de manière opératoire à l'appareil de fabrication (3), le dispositif de contrôle de fabrication (1) étant conçu pour commander le dispositif laser de fabrication (3) afin de réaliser un certain nombre d'incisions dans la cornée du donneur, les incisions dans la cornée du donneur formant, en combinaison, un motif de coupe de la cornée du donneur, la géométrie de l'implant cornéen (411) étant définie par le motif de coupe de la cornée du donneur,
le nombre d'incisions dans la cornée du donneur incluant:
- une coupe lamellaire antérieure du donneur (D-D), la coupe lamellaire antérieure du donneur (D-D) étendue dans la cornée de donneur (41) à une distance de coupe lamellaire antérieure du donneur à partir d'une surface cornéenne postérieure du donneur (41p), la coupe lamellaire antérieure du donneur (D-D) définissant une surface antérieure de l'implant cornéen (411a),
- une coupe latérale du donneur (D-C), la coupe latérale du donneur (D-C) étendue dans la cornée de donneur (41) depuis la coupe lamellaire antérieure du donneur (D-D) vers la surface cornéenne postérieure du donneur (41p) de manière circonférentiellement fermée, dans laquelle une jonction de la coupe latérale du donneur (D-C) et de la coupe lamellaire antérieure du donneur (D-D) est déplacée par rapport à un bord circonférentiel (412) de la coupe lamellaire antérieure du donneur (D-D), la coupe latérale du donneur (D-C) définissant, au moins en partie, une surface circonférentielle de l'implant cornéen, dans laquelle la coupe latérale du donneur (D-C) ne s'étend pas jusqu'à la surface cornéenne postérieure du donneur (41d),
- une coupe d'accès du donneur (D-E), la coupe d'accès du donneur (D-E) étendue depuis une périphérie, en particulier un bord circonférentiel (412), de la coupe lamellaire antérieure du donneur (D-D) jusqu'à une surface cornéenne antérieure du donneur (41a),
- une coupe lamellaire postérieure du donneur (D-B), la coupe lamellaire postérieure du donneur (D-B) étendue dans la cornée de donneur depuis un bord postérieur (413) de la coupe latérale du donneur (D-C) vers l'intérieur par rapport à la cornée de donneur (41), en particulier à une distance constante de la coupe lamellaire antérieure du donneur (D-D),
- une coupe latérale auxiliaire du donneur (D-A), la coupe latérale auxiliaire du donneur (D-A) étendue depuis un bord intérieur (414) de la coupe lamellaire postérieure du donneur (D-B) jusqu'à la surface cornéenne postérieure du donneur (41d),
le motif de coupe de la cornée de donneur définissant en outre une structure d'emboîtement de l'implant cornéen, la structure d'emboîtement de l'implant cornéen étant configurée pour interverrouiller la cornée du patient (21) avec l'implant cornéen (411) après avoir remplacé l'élément de cornée du patient (211) par l'implant cornéen (411).

11. Procédé de fabrication, à partir d'une cornée de donneur (41), d'un implant cornéen (411) adapté à l'utilisation dans une kératoplastie lamellaire postérieure, en particulier à l'aide d'un appareil de fabrication ophtalmologique (1, 2) selon la revendication 10, le procédé ne s'appliquant pas au corps humain ou animal vivant, dans lequel le procédé inclut réaliser, à l'aide d'un dispositif laser, en particulier un dispositif laser ophtalmologique (3), d'un certain nombre d'incisions dans la cornée de donneur, les incisions dans la cornée de donneur formant, en combinaison, un motif de coupe dans la cornée de donneur, la géométrie de l'implant cornéen (411) étant définie par le motif de coupe dans la cornée de donneur,
le nombre d'incisions dans la cornée de donneur incluant:
- une coupe lamellaire antérieure du donneur (D-D), la coupe lamellaire antérieure du donneur (D-D) étendue dans la cornée de donneur (41) à une distance de coupe lamellaire antérieure du donneur par rapport à la surface cornéenne antérieure du donneur (41p), la coupe lamellaire antérieure du donneur (D-D) définissant une surface antérieure de l'implant cornéen (411a);
- une coupe latérale du donneur (D-C), la coupe latérale du donneur (D-C) étendue dans la cornée de donneur (41) depuis la coupe lamellaire antérieure du donneur (D-D) vers la surface cornéenne postérieure du donneur (41p) de manière circonférentiellement fermée, dans laquelle une jonction entre la coupe latérale du donneur (D-C) et la coupe lamellaire antérieure du donneur (D-D) est déplacée par rapport à un bord circonférentiel (412) de la coupe lamellaire antérieure du donneur (D-D), la coupe latérale du donneur (D-C) définissant, au moins en partie, une surface circonférentielle de l'implant cornéen, dans laquelle la coupe latérale du donneur (D-C) ne s'étend pas jusqu'à la surface cornéenne postérieure du donneur (41d),
- une coupe d'accès du donneur (D-E), la coupe d'accès du donneur (D-E) étendue depuis une périphérie, en particulier un bord circonférentiel, de la coupe lamellaire antérieure du donneur (D-D) jusqu'à une surface cornéenne antérieure du donneur (41a),
- une coupe lamellaire postérieure du donneur (D-B), la coupe lamellaire postérieure du donneur (D-B) étendue dans une cornée de donneur depuis un bord postérieur (413) de la coupe latérale du donneur (D-C) vers l'intérieur par rapport à la cornée de donneur (41), en particulier à une distance constante de la coupe lamellaire antérieure du donneur (D-D),
- une coupe latérale auxiliaire du donneur (D-A), la coupe latérale auxiliaire du donneur (D-A) étendue depuis un bord intérieur (414) de la coupe lamellaire postérieure du donneur (D-B) jusqu'à la surface cornéenne postérieure du donneur (41d),
le motif de coupe de la cornée de donneur définissant en outre une structure d'emboîtement de l'implant cornéen, la structure d'emboîtement de l'implant cornéen étant configurée pour interverrouiller la cornée du patient (21) avec l'implant cornéen (411) après avoir remplacé l'élément de cornée du patient (211) par l'implant cornéen (411).
